# FASCICULE DE BREVET EUROPEEN

(11) **EP 1 215 213 B1**
(45) Date de publication et mention de la délivrance du brevet: **17.01.2007**
(21) Numéro de dépôt: 01403227.0
(22) Date de dépôt: 13.12.2001
(51) Int. Cl.: C07K 5/078, A61K 38/05, A61P 7/02

(54) **Dérivés bicycliques d'amino-pyrazones, leur procédé de préparation et les compositions pharmaceutiques qui les contiennent**
Bizyklische Amino-Pyrazon Derivate, deren Herstellung und pharmazeutische Zusammensetzungen
Bicyclic amino-pyrazone derivatives, their preparation and pharmaceutical compositions containing them

(30) Priorité: 14.12.2000 FR 0016321
(43) Date de publication de la demande: 19.06.2002
(73) Titulaire: Les Laboratoires Servier, 92200 Neuilly-sur-Seine (FR)
(72) Inventeur: de Nanteuil, Guillaume, 92150 Suresnes (FR); Gloanec, Philippe, 78160 Marly le Roi (FR); Verbeuren, Tony, 78540 Vernouillet (FR); Rupin, Alain, 37510 Savonniers (FR); Vallez, Marie-Odile, 77420 Champs sur Marne (FR)

(56) Documents cités:
- EP-A- 1 069 132
- WO-A-00/26210
- WO-A-00/75134
- WO-A-96/19483
- WO-A-98/17274

## Description

La présente invention concerne des nouveaux dérivés bicycliques d'amino-pyrazinones, leur procédé de préparation, les compositions pharmaceutiques qui les contiennent ainsi que leur utilisation en tant qu'inhibiteurs de protéases à sérine apparentées à la trypsine.

L'une de ces protéases à sérine, la thrombine, est l'enzyme clé de la coagulation et joue un rôle central dans la pathologie des thromboses veineuses et artérielles, en raison notamment de son fort pouvoir d'auto-amplification de la cascade de la coagulation (F. Toti et coll., Sang, Thrombose, Vaisseaux 1992, 4, 483-494 et T.M. Reilly et coll., Blood Coagulation and Fibrinolysis 1992, 3, 513-517).

L'inhibition directe et spécifique de la thrombine est plus efficace et présente moins de risques d'hémorragie que le traitement par l'héparine. Il existe actuellement des inhibiteurs directs de thrombine, mais ces substances peptidiques présentent l'inconvénient de ne pas être actives par voie orale.

Des dérivés peptidomimétiques, présentant une activité anti-thrombotique orale, ont déjà été décrits dans la littérature. C'est le cas notamment des dérivés de l'acide boronique décrits dans les brevets EP 293 881, EP 471 651, EP 615 978 et EP 792 883 et des dérivés décrits dans les brevets WO 94 29336, WO 95 23609, WO 00 26210 et EP 10 69132.

Il était donc particulièrement intéressant de synthétiser de nouveaux inhibiteurs de protéases à sérine afin d'augmenter la puissance et la sélectivité des composés déjà décrits dans la littérature.

L'activité de ces nouveaux composés est objectivée par l'augmentation de différents temps de coagulation.

De plus, ces composés sont actifs par voie orale.

Plus spécifiquement, la présente invention concerne les composés de formule (I) : 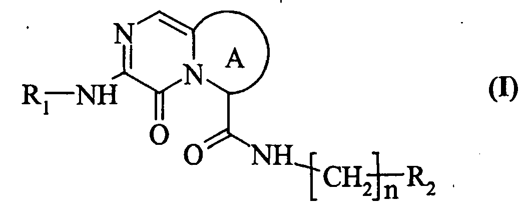 dans laquelle :
* R₁ représente un atome d'hydrogène ou un groupement alkyle (C₁-C₆) linéaire ou ramifié éventuellement substitué par un ou plusieurs groupements, identiques ou différents, choisis parmi aryle, hétéroaryle, cycloalkyle et hétérocycloalkyle,
* 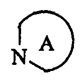 représente un cycle saturé de 4 à 7 chaînons pouvant contenir, en plus de l'atome d'azote, un ou deux hétéroatomes choisis parmi O, S et groupements -NR₃,
   dans lequel R₃ représente un atome d'hydrogène ou un groupement alkyle (C₁-C₆) linéaire ou ramifié,
* n représente un entier tel que 1 ≤ n ≤ 6,
* R₂ représente l'un quelconque des groupements suivants :
   a : 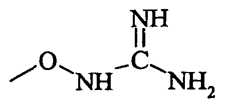
   b: 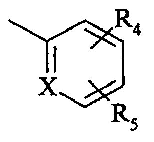 dans lequel :
      ✔ X représente un groupement CH ou un atome d'azote,
      ✔ R₄ représente un atome d'hydrogène ou d'halogène
      ✔ R₅ représente l'un quelconque des groupements :
         ◆ RₐNHCOHN- dans lequel Rₐ représente un atome d'hydrogène ou un groupement alkyle (C₁-C₆) linéaire ou ramifié,
         ◆ R_{b}SO₂NHCO(NH)ₚ-, dans lequel R_{b} représente un groupement alkyle (C₁-C₆) linéaire ou ramifié et p représente 0 ou 1,
         ◆ HON = C(NH₂)-, HN=C(NHOH)-,
         ◆ R_{c}-(CH₂)ₘ-Y- dans lequel :
            Y représente CH_{2,} O, S ou RₐN,
            m représente un nombre entier tel que 0 ≤ m ≤ 3,
            R_{c} représente un hétérocycle à 5 ou 7 chaînons, saturé ou insaturé contenant de 1 à 4 hétéroatomes choisis parmi oxygène, azote ou soufre, ledit hétérocycle contenant éventuellement une ou plusieurs fonctions carbonyles et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (lui-même éventuellement substitué par un ou deux groupements alkyle (C₁-C₆) linéaire ou ramifié),
      ou c : un système bicyclique de formule 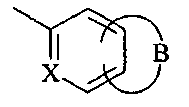 dans laquelle :
      X est tel que défini précédemment et B, avec les atomes de carbone auquel il est attaché, forme un hétérocycle à 5 ou 7 chaînons, saturé ou insaturé, contenant de 1 à 4 hétéroatomes choisis parmi oxygène, azote ou soufre, ledit hétérocycle contenant au moins une fonction carbonyle et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (éventuellement substitué par un ou deux groupements alkyle (C₁-C₆) linéaire ou ramifié),
   leurs isomères, leurs N-oxydes ainsi que leurs sels d'addition à un acide ou à une base pharmaceutiquement acceptables.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, etc. citrique, ascorbique, oxalique, méthanesulfonique, benzènesulfonique, camphorique, etc.

Parmi les bases pharmaceutiquement acceptables, on peut citer à titre non limitatif l'hydroxyde de sodium, l'hydroxyde de potassium, la triéthylamine, la tertbutylamine, etc.

Par groupement aryle, on entend phényle, biphénylyle ou naphtyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy, amino (lui-même éventuellement substitué par un ou deux groupements alkyle (C₁-C₆) linéaire ou ramifié) ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, trihalogénoalkoxy (C₁-C₆) linéaire ou ramifié, amino (éventuellement substitué par un ou deux groupements alkyle (C₁-C₆) linéaire ou ramifié), alkylcarbonyloxy (C₁-C₆) linéaire ou ramifié, carboxyméthoxy et carbamoylméthoxy (éventuellement N-substitué par un ou deux groupements alkyle (C₁-C₆) linéaire ou ramifié).

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié (éventuellement substitué par un groupement hydroxy, carboxy, amino (lui-même éventuellement substitué par un ou deux groupements alkyle (C₁-C₆) linéaire ou ramifié) ou carbamoyle (lui-même éventuellement substitué par un ou deux groupements alkyle (C₁-C₆) linéaire ou ramifié)), hydroxy, alkoxy (C₁-C₆) linéaire ou ramifié, trihalogénoalkyle (C₁-C₆) linéaire ou ramifié, phényle, amino (éventuellement N-substitué par un ou plusieurs groupements alkyle (C₁-C₆) linéaire ou ramifié), carboxyméthoxy et carbamoylméthoxy (éventuellement substitué par un ou deux groupements alkyle (C₁-C₆) linéaire ou ramifié).
Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, pyrimidinyle, pyrazinyle, pyridazinyle.

Par groupement cycloalkyle, on entend un groupement hydrocarboné mono- ou bicyclique, saturé ou insaturé, de 3 à 12 chaînons, étant entendu que le cycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié) et aryle.
Parmi les groupements cycloalkyle, on peut citer à titre non limitatif les groupements cyclopentyle, cyclohexyle, indanyle, tétrahydronaphtyle.

Par groupement hétérocycloalkyle, on entend un groupement mono- ou bicyclique, saturé ou insaturé, de 4 à 12 chaînons contenant un, deux ou trois hétéroatomes choisis parmi oxygène, azote ou soufre, étant entendu que hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié), aryle et diarylméthyle.
Parmi les groupements hétérocycloalkyle, on peut citer à titre non limitatif les groupements azétidinyle, pyrrolidinyle, pipéridyle, dihydrocyclopenta[b]pyridyle.

Les composés préférés de formule (I) sont ceux pour lesquels n est égal à 1. Le cycle 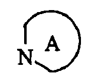 tel que défini dans la formule (I) est préférentiellement un groupement pyrrolidinyle.
Lorsque 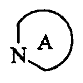 représente un cycle pyrrolidine, la configuration de ce cycle est préférentiellement S.

Les groupements R₁ préférés sont les groupements alkyles (C₁-C₆) linéaires ou ramifiés substitués par un ou plusieurs groupements aryle ou hétéroaryle.

De manière encore plus préférentielle, les groupements R₁ préférés sont les groupements alkyles (C₁-C₆) linéaires ou ramifiés substitués par un ou plusieurs groupements phényle ou pyridinyle.

A titre très préférentiel, les groupements R₁ sont les groupements (2,2-diphényl)éthyle ou (2-pyridinyl)éthyle.

Lorsque R₂ représente un groupement b, X représente préférentiellement un groupement CH et R₅ préférentiellement un groupement HN=C(NHOH)- ou Rc-(CH₂)ₘ-Y. Dans ce cas, R_{c} sera de manière encore plus préférentielle un groupement pyridine, pyrrolidinone, imidazole ou imidazoline.

Lorsque R₂ représente un système bicyclique c, celui-ci sera préférentiellement tel que soit X représente un groupement CH et, dans ce cas, B représente un cycle morpholinone, isoxazole ou pyrrole, soit X représente un atome d'azote et, dans ce cas, B représente un cycle phényle.

Les composés préférés de l'invention sont :
- (6S)-*N*-{4-[amino(hydroxyimino)méthyl]benzyl}-3-[(2,2-diphényl-éthyl)amino]-4-oxo-4,6,7,8-tétrahydropyrrolo[1,2-α]pyrazine-6-carboxamide,
- (6S)-*N*-{4-[amino(hydroxyimino)méthyl]benzyl}-4-oxo-3-{(2-(2-pyridinyl)éthyl]amino}-4,6,7,8-tétrahydropyrrolo[1,2-α]pyrazine-6-carboxamide,
- (6S)-*N*-[2-({[amino(imino)méthyl]amino}oxy)éthyl]-3-[(2,2-diphényl-éthyl)amino]-4-oxo-4,6,7,8-tétrahydropyrrolo[1,2-α]pyrazine-6-carboxamide,
- (6S)-4-oxo-*N*-[(3-oxo-3,4-dihydro-2*H*-benzoxazin-8-yl)méthyl]-3-{[2-(2-pyridinyl)éthyl]amino}-4,6,7,8-tétrahydropyrrolo[1,2-α]pyrazine-6-carboxamide,
- (6S)-4-oxo-3-{[2-(2-pyridinyl)éthyl]amino}-*N*-[2-(2-pyridinyl-méthoxy)benzyl]4,6,7,8-tétrahydropyrrolo[1,2-α]pyrazine-6-carboxamide,
- (6S)-4-oxo-*N*-{2-[(2-oxo-3-pyrrolidinyl)oxy]benzyl}-3-{[2-(2-pyridinyl)éthyl]amino}-4,6,7,8-tétrahydropyrrolo[1,2-α]pyrazine-6-carboxamide,
- (6S)-*N*-[2-(4,5-dihydro-1*H*-imidazol-2-ylméthoxy)benzyl]-4-oxo-3-{[2-(2-pyridinyl)éthyl]amino}-4,6,7,8-tétrahydropyrrolo[1,2-α]pyrazine-6-carboxamide,
- (6S)-*N*-[2-(1*H*-imidazol-2-ylméthoxy)benzyl]-4-oxo-3-{[2-(2-pyridinyl)éthyl]amino}-4,6,7,8-tétrahydropyrrolo[1,2-α]pyrazine-6-carboxamide,
- (6S)-*N*-[1*H*-indol-6-ylméthyl)-4-oxo-3-{[2-(2-pyridinyl)éthyl]amino}-4,6,7,8-tétrahydropyrrolo[1,2-α]pyrazine-6-carboxamide

L'invention s'étend également au procédé de préparation des composés de formule (I) caractérisé en ce que l'on réduit un composé de formule (II) : 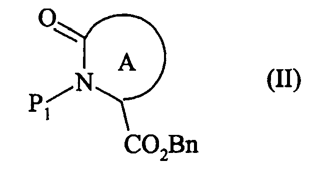 dans laquelle A a la même signification que dans la formule (I), P₁ représente un groupement protecteur de la fonction amino et Bn représente le groupement benzyle,
à l'aide d'un agent réducteur approprié.
pour conduire au composé de formule (III) : 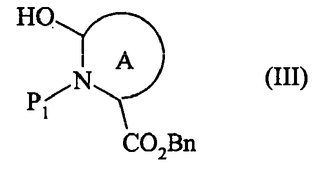 dans laquelle A, P₁ et Bn ont la même signification que précédemment,
composé de formule (III) dont on transforme la fonction hydroxy en méthoxy puis en fonction cyano par des réactions classiques de la chimie organique, pour conduire, après déprotection de la fonction amino, au composé de formule (IV) : 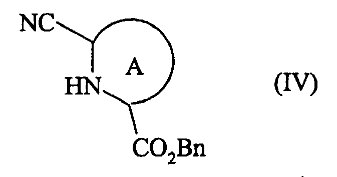 dans laquelle A et Bn ont la même signification que précédemment,
composé de formule (IV) que l'on met en réaction avec du chlorure d'oxalyle pour conduire au composé de formule (V) : 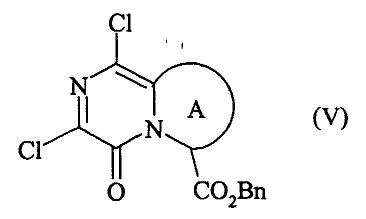 dans laquelle A et Bn ont la même signification que précédemment,
composé de formule (V) que l'on met en réaction avec un composé de formule (VI) :

R₁ - NH₂ (VI)

dans laquelle R₁ a la même signification que dans la formule (I), pour conduire au composé de formule (VII) : 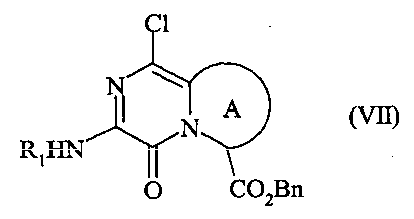 dans laquelle A, Bn et R₁ ont la même signification que précédemment,
composé de formule (VII) que l'on transforme ensuite par hydrogénation catalytique en composé de formule (VIII) : 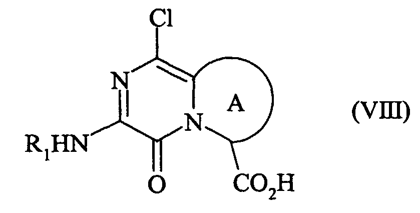 dans laquelle A et R₁ ont la même signification que précédemment,
composé de formule (VIII) que l'on transforme ensuite, par hydrogénation catalytique en milieu alcalin, en composé de formule (IX) : 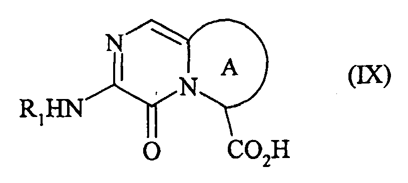 dans laquelle A et R₁ ont la même signification que précédemment,
composé de formule (IX) que l'on met en réaction avec un composé de formule (X) : 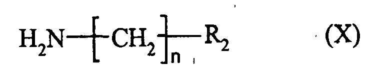 dans laquelle n et R₂ ont la même signification que dans la formule (I),
pour conduire, après éventuelle déprotection, au composé de formule (I),
composé de formule (I) que l'on transforme éventuellement en N-oxyde correspondant, que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

Les composés de formule (II) sont obtenus par benzylation des acides correspondants.

Les composés de la présente invention, outre le fait qu'ils soient nouveaux, présentent des propriétés pharmacologiques particulièrement intéressantes.

Ce sont de puissants inhibiteurs de protéases à sérine apparentées à la trypsine qui présentent une importante sélectivité vis-à-vis de la thrombine par rapport à d'autres protéases à sérine de la coagulation et de la fibrinolyse.

Ces propriétés les rendent donc utiles dans le traitement des angines stables ou non, des maladies d'origine thrombotique et/ou donnant lieu à des complications thrombotiques, dans le traitement ou la prévention de l'infarctus du myocarde et des thromboses veineuses ou artérielles, ainsi que dans le traitement des complications des maladies vasculaires et cardiovasculaires telles que l'athérosclérose, l'artérite, la maladie veineuse, et dans le traitement de toutes les maladies impliquant une formation et/ou une activité de la thrombine.

Ils peuvent également être utilisés en association thérapeutique avec un thrombolytique.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse).

### EXEMPLE 1 : (6S)-N-{4-[amino(hydroxyimino)méthyl]benzyl}-3-[2,2-diphényléthyl)amino]-4-oxo-4,6,7,8-tétrahydropyrrolo[1,2-α]pyrazine-6-carboxamide

A 2,5 mmoles d'acide (6S)-3-[(2,2-diphényléthyl)amino]-4-oxo-4,6,7,8-tétrahydropyrrolo [1,2-a]pyrazine-6-carboxylique obtenu selon le procédé décrit dans la demande FR 9907538 dans 50 ml de diméthylformamide anhydre sont ajoutés 0,9 g de chlorhydrate de 4-(aminométhyl)-*N*'-hydroxybenzènecarboximidamide obtenu selon le procédé décrit dans Synth. Comm. (28 (23), 4419-4429, 1998), 1,3 ml de diisopropyléthylamine et 0,4 g d'hydroxybenzotriazole. Après solubilisation, 0,9 g de tétrafluoroborate de O-benzotriazolyl-tétraméthylisouronium sont ajoutés et l'ensemble est maintenu une nuit sous agitation. Après filtration et évaporation, le résidu est repris par de l'acétate d'éthyle. La phase organique est lavée, séchée, filtrée et évaporée. Le produit attendu est obtenu sous forme solide après purification du résidu par chromatographie sur gel de silice en utilisant comme éluant un mélange dichlorométhane/méthanol (95/5).
*Spectre de masse : [MH]*⁺ *m*/*z* = *522*

### EXEMPLE 2 : (6S)-N-{4-[amino(hydroxyimino)méthyl]benzyl}-4-oxo-3-{(2-(2-pyridinyl)éthyl]amino}-4,6,7,8-tétrahydropyrrolo[1,2-α]pyrazine-6-carboxamide

Le produit attendu est obtenu selon le procédé décrit dans l'exemple 1 en remplaçant l'acide (6S)-3-[(2,2-diphényléthyl)amino]-4-oxo-4,6,7,8-tétrahydropyrrolo[1,2-α]pyrazine-6-carboxylique par l'acide (6S)-3-{[2-(2-pyridinyl)éthyl]amino}-4-oxo-4,6,7,8-tétrahydropyrrolo-[1,2-α]pyrazine-6-carboxylique.
*Microanalyse élémentaire :*

| | *C%* | *H%* | *N%* |
|---|---|---|---|
| *Calculé* | *61,73* | *5, 63* | *21, 91* |
| *Trouvé* | *62, 03* | *5,50* | *21, 78* |

### EXEMPLE 3 : (6S)-N-[2-({[amino(imino)méthyl]amino}oxy)éthyl]-3-[(2,2-diphényléthyl)amino]-4-oxo-4,6,7,8-tétrahydropyrrolo[1,2-α]pyrazine-6-carboxamide dichlorhydrate

### Stade A : (6S)-N-[2-({[(tertbutyloxycarbonylamino)(tertbutyloxycarbonylimino)méthyl]amino}oxy)éthyl]-3-[(2,2-diphényléthyl)amino]-4-oxo-4,6,7,8-tétrahydropyrrolo[1,2-a]pyrazine-6-carboxamide

Le produit attendu est obtenu selon le procédé décrit dans l'exemple 1 en remplaçant le chlorhydrate de 4-(aminométhyl)-*N'*-hydroxybenzènecarboximidamide par la *N*-(2-aminoéthoxy)-*N',N*''-(ditertbutyloxycarbonyl)guanidine

### Stade B : (6S)-N-[2-({[amino(imino)méthyl)amino}oxy)éthyl]-3-[(2,2-diphényl-éthyl) amino]-4-oxo-4,6,7,8-tétrahydropyrrolo[1,2-a]pyrazine-6-carboxamide dichlorhydrate

A 1,8 mmoles du composé décrit au stade précédent dans 20 ml de dichlorométhane anhydre, sont ajoutés 10 ml d'une solution d'acide chlorhydrique 4M dans le dioxane. L'ensemble est agité une nuit à température ambiante. Après évaporation des solvants, le résidu est repris par de l'eau. La solution est filtrée et lyophilisée et conduit au produit attendu.
*Spectre de masse : [M+H⁺] m*/*z = 475*

Les exemples suivants ont été préparés selon le procédé décrit dans l'exemple 1 en utilisant les produits de départ correspondants.

### EXEMPLE 4 : (6S)-4-oxo-N-[(2-oxo-2,3-dihydro-1H-pyrrolo[2,3-c]pyridin-5-yl) méthyl]-3-{[2-(2-pyridinyl)éthyl]amino}-4,6,7,8-tétrahydropyrrolo [1,2-α]pyrazine-6-carboxamide

### EXEMPLE 5 : (6S)-4-oxo-N-[(2-oxo-2,3-dihydro-1H-imidazo[4,5-c]pyridin-6-yl) méthyl]-3-{[2-(2-pyridinyl)éthyl]amino}-4,6,7,8-tétrahydropyrrolo [1,2-α]pyrazine-6-carboxamide

### EXEMPLE 6 : (6S)-4-oxo-N-[(2-oxo-2,3-dihydro-1H-benzimidazol-4-yl)méthyl]-3-{[2-(2-pyridinyl)éthyl]amino}-4,6,7,8-tétrahydropyrrolo[1,2-α] pyrazine-6-carboxamide

### EXEMPLE 7 : (6S)-4-oxo-N-[(2-oxa-2,3-dihydro-1H-benzimidazol-5-yl)méthyl]-3-{[2-(2-pyridinyl)éthyl]amino}-4,6,7,8-tétrahydropyrrolo[1,2-α] pyrazine-6-carboxamide

### EXEMPLE 8 : (6S)-4-oxo-N-[(3-oxo-3,4-dihydro-2H-benzoxazin-8-yl)méthyl]-3-{[2-(2-pyridinyl)éthyl]amino}-4,6,7,8-tétrahydropyrrolo[1,2-α]pyrazine-6-carboxamide

### EXEMPLE 9 : (6S)-4-oxo-N[(4-oxo-3,4-dihydro-2H-1,3-benzoxazin-8-yl)méthyl]-3-{[2-(2-pyridinyl)éthyl]amino}-4,6,7,8-tétrahydropyrrolo[1,2-α] pyrazine-6-carboxamide

### EXEMPLE 10 : (6S)-4-oxo-3-{[2-(2-pyridinyl)éthyl]amino}-N-[2-(2-pyridinylméthoxy)benzyl]-4,6,7,8-tétrahydropyrrolo[1,2-α]pyrazine-6-carboxamide

### EXEMPLE 11 : (6S)-N-{2-[(2,4-dioxo-1,3-thiazolidin-5-yl)méthyl]benzyl}-4-oxo-3-{[2-(2-pyridinyl)éthyl]amino}-4,6,7,8-tétrahydropyrrolo[1,2-α]pyrazine-6-carboxamide

### EXEMPLE 12 : (6S)-N-{2-[(2,4-dioxo-1,3-thiazolidin-5-yl)oxy]benzyl}-4-oxo-3-{[2-(2-pyridinyl)éthyl]amino}-4,6,7,8-tétrahydropyrrolo[1,2-α]pyrazine-6-carboxamide

### EXEMPLE 13 : (6S)-4-oxo-N-{2-[(5-oxo-4,5-dibydro-3H-1,2,4-triazol-3-yl)oxy] benzyl}-3-{[2-(2-pyridinyl)éthyl]amino}-4,6,7,8-tétrahydropyrrolo [1,2-α]pyrazine-6-carboxamide

### EXEMPLE 14 : (6S)-4-oxo-N-{2-[(2-oxo-3-Pyrrolidinyl)oxy]benzyl}-3-([2-(2-pyridinyl)éthyl]amino}-4,6,7,8-tétrahydropyrrolo[1,2-α]pyrazine-6-carboxamide

### EXEMPLE 15 : (6S)-N-[2-(4,5-dibydro-1H-imidazol-2-ylméthoxy)benzyl]-4-oxo-3-{[2-(2-pyridinyl)éthyl]amino}-4,6,7,8-tétrahydropyrrolo[1,2-a]pyrazine-6-carboxamide

### EXEMPLE 16 : (6S)-N-[2-(1H-imidazol-2-ylméthoxy)benzyl]-4-oxo-3-{[2-(2-pyridinyl)éthyl]amino}-4,6,7,8-tétrahydropyrrolo[1,2-α]pyrazine-6-carboxamide

### ETUDE PHARMACOLOGIQUE DES DERIVES DE L'INVENTION

### EXEMPLE A : Inhibition de la thrombine et de protéases à sérine de la fibrinolyse

Pour évaluer in vitro l'activité inhibitrice des produits de l'invention sur la thrombine humaine (Sigma, activité spécifique 3230 UNIH/mg), le fibrinogène humain purifié (4 mM, Stago) (Fg) a été ajouté à une quantité donnée de thrombine (0.7 nM) préalablement incubée avec ou sans l'inhibiteur à tester (20°C, 10 minutes).

Pour évaluer in vitro la sélectivité de ces produits vis-à-vis de la plasmine, le même protocole a été appliqué à la plasmine humaine purifiée (2 nM, Stago), en utilisant pour substrat un peptide paranitroanilidé : <Glu-Phe-Lys-pNA (0.50 mM, S 2403, Kabi).

Inhibiteurs, enzymes et substrats sont dilués dans le même tampon (tampon phosphate 0.01 mM, pH 7.4, contenant 0.12 M de chlorure de sodium et 0.05 % de sérum albumine bovine) puis distribués dans une microplaque en polystyrène sous un volume de 50 µl.

La fibrine formée par la thrombine ou par le paranitroanilide libéré par l'action de la protéase à sérine est mesurée spectrophotométriquement à 405 nm après 15 à 30 minutes de réaction à 20°C.

Lors de ce test, il a été montré notamment que la concentration en nM inhibant 50 % de l'activité enzymatique (CI₅₀) de la thrombine par rapport au contrôle sans produit est égale à 141 nM pour le composé de l'exemple 1,313 nM pour le composé de l'exemple 2, 4 nM pour le composé de l'exemple 3, 136 nM pour le composé de l'exemple 10, 12 nM pour le composé de l'exemple 15, 140 nM pour le composé de l'exemple 17 et 22 nM pour le composé de l'exemple 18.

Les composés de l'invention sont, d'autre part, très sélectifs vis-à-vis de la thrombine par rapport à d'autres protéases à sérine de la fibrinolyse (plasmine, tPa et uPa).

### EXEMPLE B : Activité anti-coagulante, mesure de temps de thrombine et temps de céphaline activée chez l'homme

Afin d'évaluer l'activité anti-coagulante des composés de l'invention, le temps de thrombine (TT) et le temps de céphaline activée (TCA) ont été déterminés dans des échantillons de plasma humain. Un coagulomètre ST₄ a été utilisé. Un plasma, pauvre en plaquettes, lyophilisé, est repris dans de l'eau distillée. Le TT est réalisé avec le réactif Thrombine Prest et le TCA avec le réactif Céphaline PTT Automate.
L'inhibiteur ou le solvant (10 µl) est additionné au plasma (90 µl), puis incubé 2 minutes à 37°C. 100 µl de Thrombine Prest (TT) ou de Céphaline PTT Automate (TCA) sont ajoutés en déclenchant le chronomètre.

Dans ces conditions, le TT est de l'ordre de 18 secondes, le TCA est de l'ordre de 12 secondes. L'activité d'un antagoniste est évaluée par sa capacité de prolonger le TT et le TCA par rapport au témoin. L'effet des inhibiteurs est exprimé par la concentration en µM qui multiplie de 2 le temps de coagulation (Ctt₂).

Les composés de l'invention ont produit des prolongations des temps de coagulation très importantes et certains Ctt₂ sont exemplifiés dans le tableau 1 ci-dessous :

**Tableau 1**

| *Exemple* | *TT Ctt₂ (µM)* | *TCA Ctt₂ (µM)* |
|---|---|---|
| 2 | 3,6 | 20 |
| 3 | 0,10 | 1,1 |
| 15 | 0,19 | 1,91 |
| 18 | 0,28 | 3,07 |

### EXEMPLE DE COMPOSITION PHARMACEUTIQUE :

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 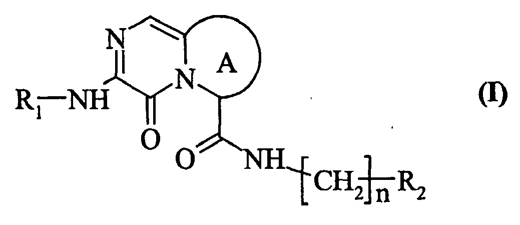 dans laquelle :
* R₁ représente un atome d'hydrogène ou un groupement alkyle (C₁-C₆) linéaire ou ramifié éventuellement substitué par un ou plusieurs groupements, identiques ou différents, choisis parmi aryle, hétéroaryle, cycloalkyle et hétérocycloalkyle,
* 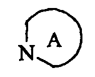 représente un cycle saturé de 4 à 7 chaînons pouvant contenir, en plus de l'atome d'azote, un ou deux hétéroatomes choisis parmi O et S, et groupements -NR₃,
dans lequel R₃ représente un atome d'hydrogène ou un groupement alkyle (C₁-C₆) linéaire ou ramifié,
* n représente un entier tel que 1 ≤ n ≤ 6,
* R₂ représente l'un quelconque des groupements suivants :
a : 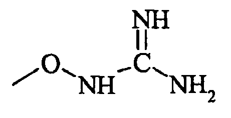
b: 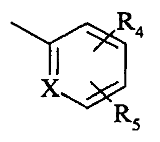 dans lequel :
✔ X représente un groupement CH ou un atome d'azote,
✔ R₄ représente un atome d'hydrogène ou d'halogène
✔ R₅ représente l'un quelconque des groupements :
◆ RₐNHCOHN- dans lequel Rₐ représente un atome d'hydrogène ou un groupement alkyle (C₁-C₆) linéaire ou ramifié,
◆ R_{b}SO₂NHCO(N-H)ₚ-, dans lequel R_{b} représente un groupement alkyle (C₁-C₆) linéaire ou ramifié et p représente 0 ou 1,
◆ HON = C(NH₂)-, HN=C(NHOH)-,
◆ R_{c}-(CH₂)ₘ-Y- dans lequel :
Y représente CH₂, O, S ou RₐN,
m représente un nombre entier tel que 0 ≤ m ≤ 3,
R_{c} représente un hétérocycle à 5 ou 7 chaînons, saturé ou insaturé contenant de 1 à 4 hétéroatomes choisis parmi oxygène, azote ou soufre, ledit hétérocycle contenant éventuellement une ou plusieurs fonctions carbonyles et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (lui-même éventuellement substitué par un ou deux groupements alkyle (C₁-C₆) linéaire ou ramifié),
ou c : un système bicyclique de formule 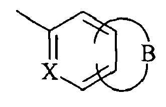 dans laquelle:
X est tel que défini précédemment et B, avec les atomes de carbone auquel il est attaché, forme un hétérocycle à 5 ou 7 chaînons, saturé ou insaturé, contenant de 1 à 4 hétéroatomes choisis parmi oxygène, azote ou soufre, ledit hétérocycle contenant au moins une fonction carbonyle et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (éventuellement substitué par un ou deux groupements alkyle (C₁-C₆) linéaire ou ramifié),
leurs isomères, leurs N-oxydes ainsi que leurs sels d'addition à un acide ou à une base pharmaceutiquement acceptables,
étant entendu que par groupement aryle, on entend phényle, biphénylyle ou naphtyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy, amino (lui-même éventuellement substitué par un ou deux groupements alkyle (C₁-C₆) linéaire ou ramifié) ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, trihalogénoalkoxy (C₁-C₆) linéaire ou ramifié, amino (éventuellement substitué par un ou deux groupements alkyle (C₁-C₆) linéaire ou ramifié), alkylcarbonyloxy (C₁-C₆) linéaire ou ramifié, carboxyméthoxy et carbamoylméthoxy (éventuellement N-substitué par un ou deux groupements alkyle (C₁-C₆) linéaire ou ramifié),
par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié (éventuellement substitué par un groupement hydroxy, carboxy, amino (lui-même éventuellement substitué par un ou deux groupements alkyle (C₁-C₆) linéaire ou ramifié) ou carbamoyle (lui-même éventuellement substitué par un ou deux groupements alkyle (C₁-C₆) linéaire ou ramifié)), hydroxy, alkoxy (C₁-C₆) linéaire ou ramifié, trihalogénoalkyle (C₁-C₆) linéaire ou ramifié, phényle, amino (éventuellement N-substitué par un ou plusieurs groupements alkyle (C₁-C₆) linéaire ou ramifié), carboxyméthoxy et carbamoylméthoxy (éventuellement substitué par un ou deux groupements alkyle (C₁-C₆) linéaire ou ramifié),
par groupement cycloalkyle, on entend un groupement hydrocarboné mono- ou bicyclique, saturé ou insaturé, de 3 à 12 chaînons, étant entendu que le cycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié) et aryle,
par groupement hétérocycloalkyle, on entend un groupement mono- ou bicyclique, saturé ou insaturé, de 4 à 12 chaînons contenant un, deux ou trois hétéroatomes choisis parmi oxygène, azote ou soufre, étant entendu que l'hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié), aryle et diarylméthyle.

2. Composés de formule (I) selon la revendication 1 tels que n est égal à 1.

3. Composés de formule (I) selon la revendication 1 tels que le cycle 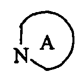 est un cycle pyrrolidinyle.

4. Composés de formule (I) selon la revendication 1 tels que R₁ représente un groupement alkyle (C₁-C₆) linéaire ou ramifié substitué par un ou plusieurs groupements aryle ou hétéroaryle.

5. Composés de formule (I) selon la revendication 4 tels que le groupement aryle est un groupement phényle.

6. Composés de formule (I) selon la revendication 4 tels que le groupement hétéroaryle est le groupement pyridinyle.

7. Composés de formule (I) selon la revendication 1 tels que R₂ représente un groupement

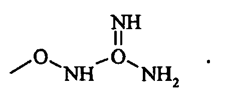

8. Composés de formule (I) selon la revendication 1 tels que R₂ représente un groupement 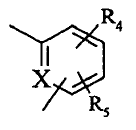 dans lequel
✔ X représente un groupement CH ou un atome d'azote,
✔ R₄ représente un atome d'hydrogène ou d'halogène
R₅ représente l'un quelconque des groupements :
◆ RₐNHCOHN- dans lequel Rₐ représente un atome d'hydrogène ou un groupement alkyle (C₁-C₆) linéaire ou ramifié,
◆ R_{b}SO₂NHCO(NH)ₚ-, dans lequel R_{b} représente un groupement alkyle (C₁-C₆) linéaire ou ramifié et p représente 0 ou 1,
◆ HON = C(NH₂)-, HN=C(NHOH)-,
◆ R_{c}-(CH₂)ₘ-Y- dans lequel :
Y représente CH₂, O, S ou RₐN,
m représente un nombre entier tel que 0 ≤ m ≤ 3,
R_{c} représente un hétérocycle à 5 ou 7 chaînons, saturé ou insaturé contenant de 1 à 4 hétéroatomes choisis parmi oxygène, azote ou soufre, ledit hétérocycle contenant éventuellement une ou plusieurs fonctions carbonyles et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (éventuellement substitué par un ou deux groupements alkyle (C₁-C₆) linéaire ou ramifié).

9. Composés de formule (I) selon la revendication 1 tels que R₂ représente un système bicyclique de formule 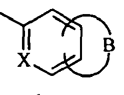 dans lequel X représente un groupement CH ou un atome d'azote, et B, avec les atomes de carbone auquel il est attaché, forme un hétérocycle à 5 ou 7 chaînons, saturé ou insaturé, contenant de 1 à 4 hétéroatomes choisis parmi oxygène, azote ou soufre, ledit hétérocycle contenant au moins une fonction carbonyle et éventuellement substitué, par un ou plusieurs, identiques ou différents, atomes d'halogène, groupements alkyle (C₁-C₆) linéaire ou ramifié, alkoxy (C₁-C₆) linéaire ou ramifié ou amino (éventuellement substitué par un ou deux groupements alkyle (C₁-C₆) linéaire ou ramifié).

10. Composé de formule (I) selon la revendication 1 qui est le (6S)-*N*-{4-[amino(hydroxyimino)méthyl]benzyl}-3-[(2,2-diphényl-éthyl)amino]-4-oxo-4,6,7,8-tétrahydropyrrolo[1,2-α]pyrazine-6-carboxamide, ses isomères, ses N-oxyde ainsi que ses sels d'addition à un acide ou à une base pharmaceutiquement acceptables.

11. Composé de formule (I) selon la revendication 1 qui est le (6S)-*N*-{4-[amino(hydroxyimino)méthyl]benzyl}-4-oxo-3-{(2-(2-pyridinyl)éthyl]amino}-4,6,7,8-tétrahydropyrrolo[1,2-α]pyrazine-6-carboxamide, ses isomères, ses N-oxyde ainsi que ses sels d'addition à un acide ou à une base pharmaceutiquement acceptables.

12. Composé de formule (I) selon la revendication 1 qui est le (6S)-*N*-[2-({[amino(imino)méthyl]amino}oxy)éthyl]-3-[(2,2-diphényléthyl)amino]-4-oxo-4,6,7,8-tétrahydropyrrolo[1,2-α]pyrazine-6-carboxamide, ses isomères, ses N-oxyde ainsi que ses sels d'addition à un acide ou à une base pharmaceutiquement acceptables.

13. Composé de formule (I) selon la revendication 1 qui est le (6S)-4-oxo-*N*-[(3-oxo-3,4-dihydro-2*H*-benzoxazin-8-yl)méthyl]-3-{[2-(2-pyridinyl)éthyl]amino}-4,6,7,8-tétrahydropyrrolo[1,2-α]pyrazine-6-carboxamide, ses isomères, ses N-oxyde ainsi que ses sels d'addition à un acide ou à une base pharmaceutiquement acceptables.

14. Composé de formule (I) selon la revendication 1 qui est le (6S)-4-oxo-3-{[2-(2-pyridinyl)éthyl]amino}-*N*-[2-(2-pyridinyl-méthoxy)benzyl]-4,6,7,8-tétrahydropyrrolo[1,2-*α*]pyrazine-6-carboxamide, ses isomères, ses N-oxyde ainsi que ses sels d'addition à un acide ou à une base pharmaceutiquement acceptables.

15. Composé de formule (I) selon la revendication 1 qui est le (6S)-4-oxo-N-{2-[(2-oxo-3-pyrrolidinyl)oxy]benzyl}-3-{[2-(2-pyridinyl)éthyl]amino}-4,6,7,8-tétrahydropyrrolo[1,2-α] pyrazine-6-carboxamide, ses isomères, ses N-oxyde ainsi que ses sels d'addition à un acide ou à une base pharmaceutiquement acceptables.

16. Composé de formule (I) selon la revendication 1 qui est le (6S)-*N*-[2-(4,5-dihydro-1*H-*imidazol-2-ylméthoxy)benzyl]-4-oxo-3-{[2-(2-pyridinyl)éthyl]amino}-4,6,7,8-tétrahydropyrrolo[1,2-a]pyrazine-6-carboxamide, ses isomères, ses N-oxyde ainsi que ses sels d'addition à un acide ou à une base pharmaceutiquement acceptables.

17. Composé de formule (I) selon la revendication 1 qui est le (6S)-*N*-[2-(1*H*-imidazol-2-ylméthoxy)benzyl]-4-oxo-3-{[2-(2-pyridinyl)éthyl]amino}-4,6,7,8-tétrahydropyrrolo[1,2-α]pyrazine-6-carboxamide, ses isomères, ses N-oxydes ainsi que ses sels d'addition à un acide ou à une base pharmaceutiquement acceptables.

18. Procédé de préparation des composés de formule (I) selon la revendication 1 **caractérisé en ce que** l'on réduit un composé de formule (II) : 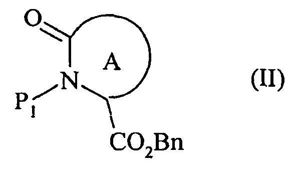 dans laquelle A a la même signification que dans la formule (I), P₁ représente un groupement protecteur de la fonction amino et Bn représente le groupement benzyle,
à l'aide d'un agent réducteur approprié,
pour conduire au composé de formule (III) : 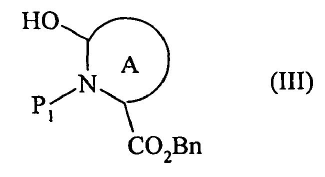 dans laquelle A, P₁ et Bn ont la même signification que précédemment,
composé de formule (III) dont on transforme la fonction hydroxy en méthoxy puis en fonction cyano par des réactions classiques de la chimie organique, pour conduire, après déprotection de la fonction amino, au composé de formule (IV) : 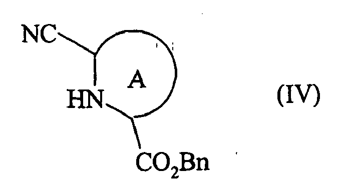 dans laquelle A et Bn ont la même signification que précédemment,
composé de formule (IV) que l'on met en réaction avec du chlorure d'oxalyle pour conduire au composé de formule (V) : 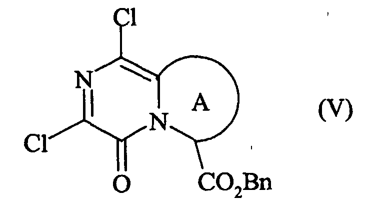 dans laquelle A et Bn ont la même.signification que précédemment,
composé de formule (V) que l'on met en réaction avec un composé de formule (VI) :
R₁ - NH₂ (VI)
dans laquelle R₁ a la même signification que dans la formule (I), pour conduire au composé de formule (VII) : 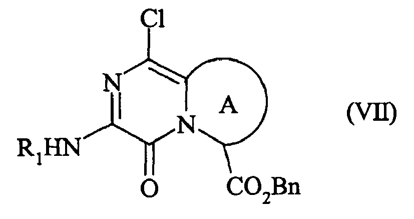 dans laquelle A, Bn et R₁ ont la même signification que précédemment,
composé de formule (VII) que l'on transforme ensuite par hydrogénation catalytique en composé de formule (VIII): 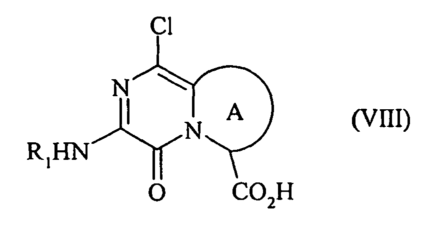 dans laquelle A et R₁ ont la même signification que précédemment,
composé de formule (VIII) que l'on transforme ensuite, par hydrogénation catalytique en milieu alcalin, en composé de formule (IX) : 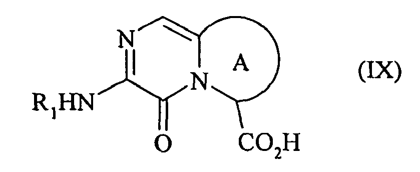 dans laquelle A et R₁ ont la même signification que précédemment,
composé de formule (IX) que l'on met en réaction avec un composé de formule (X) : 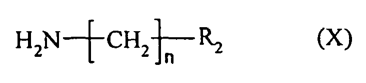 dans laquelle n et R₂ ont la même signification que dans la formule (I),
pour conduire, après éventuelle déprotection, au composé de formule (I),
composé de formule (I) que l'on transforme éventuellement en N-oxyde correspondant, que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

19. Composition pharmaceutique contenant comme principe actif un composé selon l'une quelconque des revendications 1 à 17, en combinaison avec un ou plusieurs véhicules inertes, non toxiques et pharmaceutiquement acceptables.

20. Composition pharmaceutique selon la revendication 19 utile en tant qu'inhibiteur de protéases à sérine apparentées à la trypsine.

21. Composition pharmaceutique selon la revendication 20 utile en tant qu'inhibiteur de thrombine.

## Claims

1. Compounds of formula (I) : 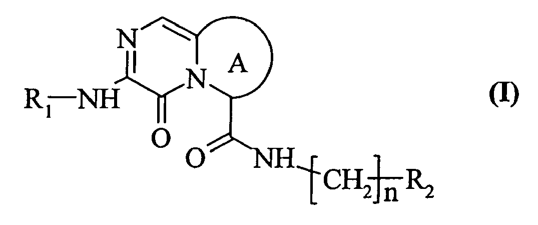 wherein
* R₁ represents a hydrogen atom, or a linear or branched (C₁-C₆)alkyl group optionally substituted by one or more identical or different groups selected from aryl, heteroaryl, cycloalkyl and heterocycloalkyl,
* 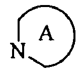 represents a saturated ring having from 4 to 7 ring members that may contain, in addition to the nitrogen atom, one or two hetero atoms selected from O and S and -NR₃ groups,
wherein R₃ represents a hydrogen atom or a linear or branched (C₁-C₆)alkyl group,
* n represents an integer such that 1 ≤ n ≤ 6,
* R₂ represents any one of the following groups :
a: 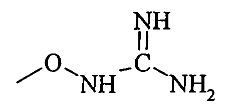
b: 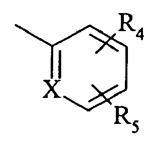 wherein :
✔ X represents a CH group or a nitrogen atom,
✔ R₄ represents a hydrogen or halogen atom,
✔ R₅ represents any one of the groups :
◆ RₐNHCOHN- wherein Rₐ represents a hydrogen atom or a linear or branched (C₁-C₆)alkyl group,
◆ R_{b}SO₂NHCO(NH)p-, wherein R_{b} represents a linear or branched (C₁-C₆)-alkyl group and p represents 0 or 1,
◆ HON = C(NH₂)-, HN=C(NHOH)-,
◆ R_{c}-(CH₂)ₘ-Y- wherein :
Y represents CH_{2,} O, S or RₐN,
m represents an integer such that 0 ≤ m ≤ 3,
R_{c} represents a saturated or unsaturated heterocycle having 5 or 7 ring members containing from 1 to 4 hetero atoms selected from oxygen, nitrogen and sulphur, the said heterocycle optionally containing one or more carbonyl functions and optionally being substituted by one or more identical or different substituents selected from halogen atoms, linear or branched (C₁-C₆)alkyl groups, linear or branched (C₁-C₆)alkoxy groups, and amino (itself optionally substituted by one or two linear or branched (C₁-C₆)alkyl groups),
or c : a bicyclic system of formula 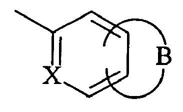 wherein :
X is as defined hereinbefore and B, together with the carbon atoms to which it is attached, forms a saturated or unsaturated heterocycle having 5 or 7 ring members containing from 1 to 4 hetero atoms selected from oxygen, nitrogen and sulphur, the said heterocycle containing at least one carbonyl function and optionally being substituted by one or more identical or different substituents selected from halogen atoms, linear or branched (C₁-C₆)alkyl groups, linear or branched (C₁-C₆)alkoxy groups, and amino (optionally substituted by one or two linear or branched (C₁-C₆)alkyl groups),
their isomers, their N-oxides and pharmaceutically acceptable addition salts thereof with an acid or a base,
there being understood by an aryl group phenyl, biphenylyl or naphthyl, each of those groups optionally being substituted by one or more identical or different groups selected from halogen, linear or branched (C₁-C₆)alkyl (optionally substituted by a hydroxy group, a carboxy group, an amino group (itself optionally substituted by one or two linear or branched (C₁-C₆)alkyl groups) or a carbamoyl group (itself optionally substituted by one or two linear or branched (C₁-C₆)alkyl groups)), linear or branched (C₁-C₆)alkoxy, hydroxy, linear or branched (C₁-C₆)trihaloalkyl, linear or branched (C₁-C₆)trihaloalkoxy, amino (optionally substituted by one or two linear or branched (C₁-C₆)alkyl groups), linear or branched (C₁-C₆)alkylcarbonyloxy, carboxymethoxy and carbamoylmethoxy (optionally N-substituted by one or two linear or branched (C₁-C₆)alkyl groups),
there being understood by a heteroaryl group an aromatic mono- or bi-cyclic group having from 5 to 12 ring members containing one, two or three hetero atoms selected from oxygen, nitrogen and sulphur, wherein the heteroaryl may optionally be substituted by one or more identical or different groups selected from halogen, linear or branched (C₁-C₆)alkyl (optionally substituted by a hydroxy group, a carboxy group, an amino group (itself optionally substituted by one or two linear or branched (C₁-C₆)alkyl groups) or a carbamoyl group (itself optionally substituted by one or two linear or branched (C₁-C₆)alkyl groups)), hydroxy, linear or branched (C₁-C₆)alkoxy, linear or branched (C₁-C₆)trihaloalkyl, phenyl, amino (optionally N-substituted by one or more linear or branched (C₁-C₆)alkyl groups), carboxymethoxy and carbamoylmethoxy (optionally substituted by one or two linear or branched (C₁-C₆)alkyl groups),
there being understood by a cycloalkyl group a saturated or unsaturated, mono- or bi-cyclic hydrocarbon group having from 3 to 12 ring members, wherein the ring may optionally be substituted by one or more identical or different groups selected from halogen, linear or branched (C₁-C₆)alkyl, linear or branched (C₁-C₆)alkoxy, hydroxy, linear or branched (C₁-C₆)trihaloalkyl, amino (optionally substituted by one or more linear or branched (C₁-C₆)alkyl groups) and aryl,
there being understood by a heterocycloalkyl group a saturated or unsaturated, mono- or bi-cyclic group having from 4 to 12 ring members containing one, two or three hetero atoms selected from oxygen, nitrogen and sulphur, wherein the heterocycle may optionally be substituted by one or more identical or different groups selected from halogen, linear or branched (C₁-C₆)alkyl, linear or branched (C₁-C₆)alkoxy, hydroxy, linear or branched (C₁-C₆)trihaloalkyl, amino (optionally substituted by one or more linear or branched (C₁-C₆)alkyl groups), aryl and diarylmethyl.

2. Compounds of formula (I) according to claim 1, wherein n is 1.

3. Compounds of formula (I) according to claim 1 wherein the ring 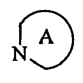 is a pyrrolidinyl ring.

4. Compounds of formula (I) according to claim 1, wherein R₁ represents a linear or branched (C₁-C₆)alkyl group substituted by one or more aryl or heteroaryl groups.

5. Compounds of formula (I) according to claim 4, wherein the aryl group is a phenyl group.

6. Compounds of formula (I) according to claim 4, wherein the heteroaryl group is a pyridyl group.

7. Compounds of formula (I) according to claim 1, wherein R₂ represents a 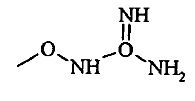 group.

8. Compounds of formula (I) according to claim 1, wherein R₂ represents a 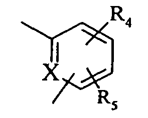 group wherein
✔ X represents a CH group or a nitrogen atom,
✔ R₄ represents a hydrogen or halogen atom,
✔ R₅ represents any one of the groups :
◆ RₐNHCOHN- wherein Rₐ represents a hydrogen atom or a linear or branched (C₁-C₆)alkyl group,
◆ R_{b}SO₂NHCO(NH)ₚ-, wherein R_{b} represents a linear or branched (C₁-C₆)-alkyl group and p represents 0 or 1,
◆ HON = C(NH₂)-, HN=C(NHOH)-,
◆ R_{c}-(CH₂)ₘ-Y- wherein :
Y represents CH₂, O, S or RₐN,
m represents an integer such that 0 ≤ m ≤ 3,
R_{c} represents a saturated or unsaturated heterocycle having 5 or 7 ring members containing from 1 to 4 hetero atoms selected from oxygen, nitrogen and sulphur, the said heterocycle optionally containing one or more carbonyl functions and optionally being substituted by one or more identical or different substituents selected from halogen atoms, linear or branched (C₁-C₆)alkyl groups, linear or branched (C₁-C₆)alkoxy groups, and amino (optionally substituted by one or two linear or branched (C₁-C₆)alkyl groups).

9. Compounds of formula (I) according to claim 1, wherein R₂ represents a bicyclic system of formula 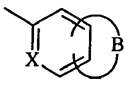 wherein X represents a CH group or a nitrogen atom, and B, together with the carbon atoms to which it is attached, forms a saturated or unsaturated heterocycle having 5 or 7 ring members containing from 1 to 4 hetero atoms selected from oxygen, nitrogen and sulphur, the said heterocycle containing at least one carbonyl function and optionally being substituted by one or more identical or different substituents selected from halogen atoms, linear or branched (C₁-C₆)alkyl groups, linear or branched (C₁-C₆)alkoxy groups, and amino (optionally substituted by one or two linear or branched (C₁-C₆)alkyl groups).

10. Compound of formula (I) according to claim 1, which is (6S)-*N*-{4-[amino(hydroxyimino)methyl]benzyl}-3-[(2,2-diphenylethyl)amino]-4-oxo-4,6,7,8-tetrahydropyrrolo[1,2-α]pyrazine-6-carboxamide, its isomers, its N-oxide and pharmaceutically acceptable addition salts thereof with an acid or a base.

11. Compound of formula (I) according to claim 1, which is (6S)-*N*-{4-[amino(hydroxyimino)methyl]benzyl}-4-oxo-3-{(2-(2-pyridyl)ethyl]amino}-4,6,7,8-tetrahydropyrrolo[1,2-α]pyrazine-6-carboxamide, its isomers, its N-oxide and pharmaceutically acceptable addition salts thereof with an acid or a base.

12. Compound of formula (I) according to claim 1, which is (6S)-*N*-[2-({[amino(imino)methyl]amino}oxy)ethyl]-3-[(2,2-diphenylethyl)amino]-4-oxo-4,6,7,8-tetrahydropyrrolo[1,2-α]pyrazine-6-carboxamide, its isomers, its N-oxide and pharmaceutically acceptable addition salts thereof with an acid or a base.

13. Compound of formula (I) according to claim 1, which is (6S)-4-oxo-*N*-[(3-oxo-3,4-dihydro-2*H*-benzoxazin-8-yl)methyl]-3-{[2-(2-pyridyl)ethyl]amino}-4,6,7,8-tetrahydropyrrolo[1,2-α]pyrazine-6-carboxamide, its isomers, its N-oxide and pharmaceutically acceptable addition salts thereof with an acid or a base.

14. Compound of formula (I) according to claim 1, which is (6S)-4-oxo-3-{[2-(2-pyridyl)ethyl]amino}-*N*-[2-(2-pyridylmethoxy)benzyl]-4,6,7,8-tetrahydropyrrolo[1,2-*α*]pyrazine-6-carboxamide, its isomers, its N-oxide and pharmaceutically acceptable addition salts thereof with an acid or a base.

15. Compound of formula (I) according to claim 1, which is (6S)-4-oxo-*N*-{2-[(2-oxo-3-pyrrolidinyl)oxy]benzyl}-3-{[2-(2-pyridyl)ethyl]amino}-4,6,7,8-tetrahydropyrrolo[1,2-α]-pyrazine-6-carboxamide, its isomers, its N-oxide and pharmaceutically acceptable addition salts thereof with an acid or a base.

16. Compound of formula (I) according to claim 1, which is (6S)-*N*-[2-(4,5-dihydro-1*H-*imidazol-2-ylmethoxy)benzyl]-4-oxo-3-{[2-(2-pyridyl)ethyl]amino}-4,6,7,8-tetrahydropyrrolo[1,2-a]pyrazine-6-carboxamide, its isomers, its N-oxide and pharmaceutically acceptable addition salts thereof with an acid or a base.

17. Compound of formula (I) according to claim 1, which is (6S)-*N*-[2-(1*H*-imidazol-2-ylmethoxy)benzyl]-4-oxo-3-{[2-(2-pyridyl)ethyl]amino}-4,6,7,8-tetrahydropyrrolo[1,2-α]pyrazine-6-carboxamide, its isomers, its N-oxide and pharmaceutically acceptable addition salts thereof with an acid or a base.

18. Process for the preparation of compounds of formula (I) according to claim 1, **characterised in that** a compound of formula (II) : 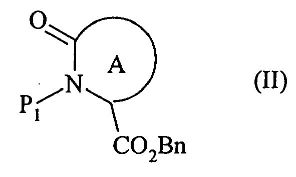 wherein A is as defined for formula (I), P₁ represents an amino function-protecting group and Bn represents a benzyl group,
is reduced using an appropriate reducing agent
to yield a compound of formula (III) : 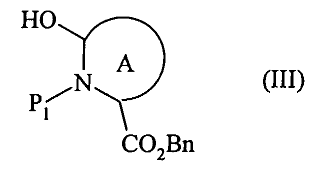 wherein A, P₁ and Bn are as defined hereinbefore,
the hydroxy function of which compound of formula (III) is converted into methoxy and then into a cyano function by conventional reactions of organic chemistry to yield, after deprotection of the amino function, a compound of formula (IV) : 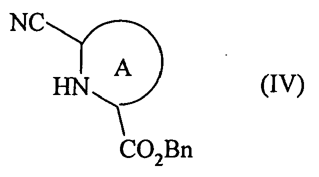 wherein A and Bn are as defined hereinbefore,
which compound of formula (IV) is reacted with oxalyl chloride to yield a compound of formula (V) : 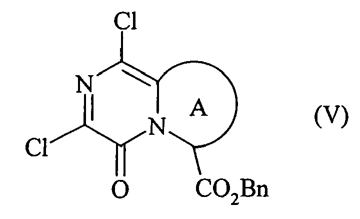 wherein A and Bn are as defined hereinbefore ,
which compound of formula (V) is reacted with a compound of formula (VI) :
R₁ - NH₂ (VI)
wherein R₁ is as defined for formula (I), to yield a compound of formula (VII) : 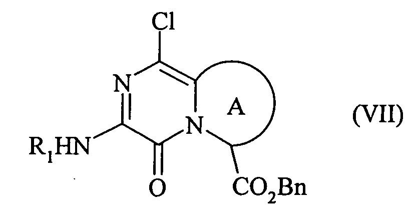 wherein A, Bn and R₁ are as defined hereinbefore ,
which compound of formula (VII) is then converted by catalytic hydrogenation into a compound of formula (VIII) : 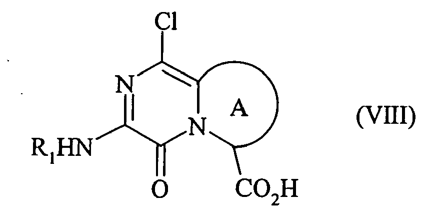 wherein A and R₁ are as defined hereinbefore ,
which compound of formula (VIII) is then converted, by catalytic hydrogenation in alkaline medium, into a compound of formula (IX) : 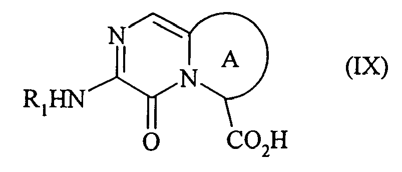 wherein A and R₁ are as defined hereinbefore ,
which compound of formula (IX) is reacted with a compound of formula (X) : 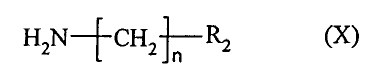 wherein n and R₂ are as defined for formula (I),
to yield, after possible deprotection, a compound of formula (I),
which compound of formula (I) is optionally converted into the corresponding N-oxide, is purified, if desired, according to a conventional purification technique, is separated, if desired, into its isomers according to a conventional separation technique, and is converted, if desired, into addition salts with a pharmaceutically acceptable acid or base.

19. Pharmaceutical composition comprising as active ingredient a compound according to any one of claims 1 to 17 in combination with one or more pharmaceutically acceptable, inert, non-toxic carriers.

20. Pharmaceutical composition according to claim 19 for use as an inhibitor of trypsin-related serine proteases.

21. Pharmaceutical composition according to claim 20 for use as a thrombin inhibitor.

## Patentansprüche

1. Verbindungen der Formel (I): 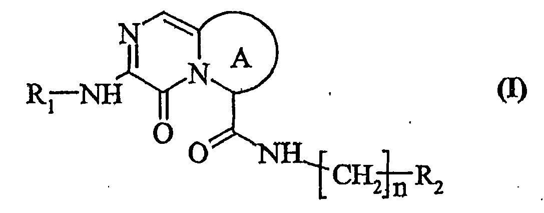 in der:
* R₁ ein Wasserstoffatom oder eine geradkettige oder verzweigte (C₁-C₆)-Alkylgruppe darstellt, die gegebenenfalls durch eine oder mehrere gleichartige oder verschiedenartige Gruppen ausgewählt aus Aryl, Heteroaryl, Cycloalkyl und Heterocycloalkyl substituiert ist,
* 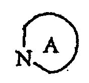 einen gesättigten Ring mit 4 bis 7 Kettengliedern bedeutet, der zuzusätzlich zu dem Stickstoffatom ein oder zwei Heteroatome ausgewählt aus O und S und Gruppen -NR₃ enthalten kann,
worin R₃ ein Wasserstoffatom oder eine geradkettige oder verzweigte (C₁-C₆)-Alkylgruppe bedeutet,
* n eine ganze Zahl bedeutet, welche die Beziehung 1 ≤ n ≤ 6 erfüllt,
* R₂ eine der folgenden Gruppen bedeutet:
a: 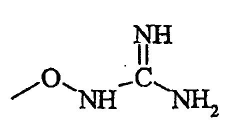
b: 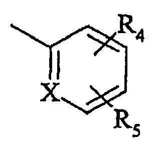 in denen:
✔ X eine Gruppe CH oder ein Stickstoffatom darstellt,
✔ R₄ ein Wasserstoffatom oder ein Halogenatom darstellt,
✔ R₅ eine der folgenden Gruppen bedeutet:
◆ RₐNHCOHN-, in der Rₐ ein Wasserstoffatom oder eine geradkettige oder verzweigte (C₁-C₆)-Alkylgruppe bedeutet,
◆ R_{b}SO₂NHCO(NH)ₚ-, in der R_{b} eine geradkettige oder verzweigte (C₁-C₆-Alkylgruppe und p 0 oder 1 bedeuten,
◆ HON = C(NH₂)-, HN=C(NHOH)-,
◆ R_{c}-(CH₂)ₘ-Y-, in der:
Y CH₂, O, S oder RₐN darstellt,
m eine ganze Zahl bedeutet, welche die Beziehung 0 ≤ m ≤ 3 erfüllt,
R_{c} einen gesättigten oder ungesättigten Heterocyclus mit 5 oder 7 Kettengliedern bedeutet, der 1 bis 4 Heteroatome ausgewählt aus Sauerstoff, Stickstoff oder Schwefel aufweist, wobei der Heterocyclus gegebenenfalls eine oder mehrere Carbonylfunktionen aufweist und gegebenenfalls durch eine oder mehrere gleichartige oder verschiedenartige Halogenatome, geradkettige oder verzweigte (C₁-C₆)-Alkylgruppen, geradkettige oder verzweigte (C₁-C₆)-Alkoxygruppen oder Aminogruppen (die ihrerseits gegebenenfalls durch eine oder zwei geradkettige oder verzweigte (C₁-C₆)-Alkylgruppen substituiert sind) substituiert ist,
oder c: ein bicyclisches System der Formel 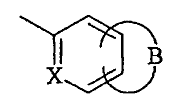 darstellt, in dem:
X die oben angegebenen Bedeutungen besitzt und B zusammen mit den Kohlenstoffatomen, an die es gebunden ist, einen gesättigten oder ungesättigten
Heterocyclus mit 5 oder 7 Kettengliedern bildet, der 1 bis 4 Heteroatome ausgewählt aus Sauerstoff, Stickstoff oder Schwefel enthält, welcher Heterocyclus mindestens eine Carbonylfunktion aufweist und gegebenenfalls durch ein oder mehrere, gleichartige oder verschiedenartige Halogenatome, geradkettige oder verzweigte (C₁-C₆)-Alkylgruppen, geradkettige oder verzweigte (C₁-C₆)-Alkoxygruppen oder Aminogruppen (die gegebenenfalls durch eine oder zwei geradkettige oder verzweigte (C₁-C₆)-Alkylgruppen substituiert sind) substituiert ist,
deren Isomere, deren N-Oxide sowie deren Additionssalze mit einer pharmazeutisch annehmbaren Säure oder Base,
wobei es sich versteht, daß man unter einer Arylgruppe Phenyl, Biphenyl oder Naphthyl versteht, wobei jede dieser Gruppen gegebenenfalls durch eine oder mehrere gleichartige oder verschiedenartige Gruppen substituiert ist, ausgewählt aus Halogen, geradkettigem oder verzweigtem (C₁-C₆)-Alkyl (gegebenenfalls substituiert durch eine Hydroxygruppe, Carboxygruppe, Aminogruppe (die ihrerseits gegebenenfalls durch eine oder zwei geradkettige oder verzweigte (C₁-C₆)-Alkylgruppen substituiert ist) oder Carbamoylgruppe (die ihrerseits gegebenenfalls durch eine oder zwei geradkettige oder verzweigte (C₁-C₆)-Alkylgruppen substituiert ist)), geradkettigem oder verzweigtem (C₁-C₆)-Alkoxy, Hydroxy, geradkettigem oder verzweigtem (C₁-C₆)-Trihalogenalkyl, geradkettigem oder verzweigtem (C₁-C₆)-Trihalogenalkoxy, Amino (gegebenenfalls substituiert durch eine oder zwei geradkettige oder verzweigte (C₁-C₆)-Alkylgruppen), geradkettigem oder verzweigtem (C₁-C₆)-Alkylcarbonyloxy, Carbomethoxy und Carbamoylmethoxy (gegebenenfalls durch eine oder zwei geradkettige oder verzweigte (C₁-C₆)-Alkylgruppen N-substituiert),
man unter einer Heteroarylgruppe eine aromatische, mono- oder bicyclische Gruppe mit 5 bis 12 Kettengliedern versteht, die ein, zwei oder drei Heteroatome ausgewählt aus Sauerstoff, Stickstoff oder Schwefel enthält, wobei es sich versteht, daß die Heteroarylgruppe gegebenenfalls substituiert sein kann durch eine oder mehrere gleichartige oder verschiedenartige Gruppen ausgewählt aus Halogen, geradkettigem oder verzweigtem (C₁-C₆)-Alkyl (gegebenenfalls substituiert durch eine Hydroxygruppe, Carboxygruppe, Aminogruppe (die ihrerseits gegebenenfalls durch eine oder zwei geradkettige oder verzweigte (C₁-C₆)-Alkylgruppen substituiert ist) oder Carbamoylgruppe (die ihrerseits gegebenenfalls durch eine oder zwei geradkettige oder verzweigte (C₁-C₆)-Alkylgruppen substituiert sein kann)), Hydroxy, geradkettigem oder verzweigtem (C₁-C₆)-Alkoxy, geradkettigem oder verzweigtem (C₁-C₆)-Trihalogenalkyl, Phenyl, Amino (gegebenenfalls durch eine oder mehrere geradkettige oder verzweigte (C₁-C₆)-Alkylgruppen N-substituiert), Carbomethoxy und Carbamoylmethoxy (gegebenenfalls substituiert durch eine oder zwei geradkettige oder verzweigte (C₁-C₆)-Alkylgruppen).
man unter einer Cycloalkylgruppe eine mono- oder bicyclische, gesättigte oder ungesättigte Kohlenwasserstoffgruppe mit 3 bis 12 Kettengliedern versteht, wobei der Ring gegebenenfalls durch eine oder mehrere gleichartige oder verschiedenartige Gruppen substituiert sein kann ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl, Amino (gegebenenfalls substituiert durch eine oder zwei geradkettige oder verzweigte (C₁-C₆)-Alkylgruppen) und Aryl,
man unter einer Heterocycloalkylgruppe eine mono- oder bicyclische, gesättigte oder ungesättigte Gruppe mit 4 bis 12 Kettengliedern versteht, die eine, zwei oder drei Heteroatome ausgewählt aus Sauerstoff, Stickstoff oder Schwefel enthält, wobei es sich versteht, daß der Heterocyclus gegebenenfalls durch eine oder mehrere gleichartige oder verschiedenartige Gruppen substituiert sein kann ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl, Amino (gegebenenfalls durch eine oder zwei geradkettige oder verzweigte (C₁-C₆)-Alkylgruppen substituiert), Aryl und Diarylmethyl.

2. Verbindungen der Formel (I) nach Anspruch 1, worin n den Wert 1 besitzt.

3. Verbindungen der Formel (I) nach Anspruch 1, worin der Ring 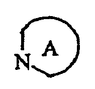 ein Pyrrolidinyl-Ring ist.

4. Verbindungen der Formel (I) nach Anspruch 1, worin R₁ eine geradkettige oder verzweigte (C₁-C₆)-Alkylgruppe darstellt, die durch eine oder mehrere Aryl- oder Heteroarylgruppen substituiert ist.

5. Verbindungen der Formel (I) nach Anspruch 4, worin die Arylgruppe eine Phenylgruppe ist.

6. Verbindungen der Formel (I) nach Anspruch 4, worin die Heteroarylgruppe die Pyridinylgruppe ist.

7. Verbindungen der Formel (I) nach Anspruch 1, worin R₂ eine Gruppe 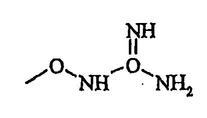 darstellt.

8. Verbindungen der Formel (I) nach Anspruch 1, worin R₂ eine Gruppe 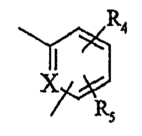 darstellt, in der
✔ X eine Gruppe CH oder ein Stickstoffatom darstellt,
✔ R₄ ein Wasserstoffatom oder ein Halogenatom darstellt,
✔ R₅ eine der folgenden Gruppen darstellt:
◆ RₐNHCOHN-, worin Rₐ ein Wasserstoffatom oder eine geradkettige oder verzweigte (C₁-C₆)-Alkylgruppe bedeutet,
◆ R_{b}SO₂NHCO(NH)ₚ-, worin R_{b} eine geradkettige oder verzweigte (C₁-C₆)-Alkylgruppe und p 0 oder 1 bedeuten,
◆ HON = C(NH₂)-, HN=C(NHOH)-,
◆ R_{c}-(CH₂)ₘ-Y-, in der:
Y CH₂, O, S oder RₐN darstellt,
m eine ganze Zahl bedeutet, welche die Beziehung 0 ≤ m s 3 erfüllt,
R_{c} einen gesättigten oder ungesättigten Heterocyclus mit 5 oder 7 Kettengliedern bedeutet, der 1 bis 4 Heteroatome ausgewählt aus Sauerstoff, Stickstoff oder Schwefel enthält, wobei der Heterocyclus gegebenenfalls eine oder mehrere Carbonylgruppen enthält und gegebenenfalls durch ein oder mehrere gleichartige oder verschiedenartige Halogenatome, geradkettige oder verzweigte (C₁-C₆)-Alkylgruppen, geradkettige oder verzweigte (C₁-C₆)-Alkoxygruppen oder Aminogruppen (die gegebenenfalls durch eine oder zwei geradkettige oder verzweigte (C₁-C₆)-Alkylgruppen substituiert sind) substituiert ist.

9. Verbindungen der Formel (I) nach Anspruch 1, worin R₂ ein bicyclisches System der Formel 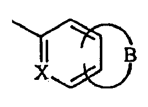 bedeutet, in der X eine Gruppe CH oder ein Stickstoffatom bedeutet und B zusammen mit den Kohlenstoffatomen, an die es gebunden ist, einen gesättigten oder ungesättigten Heterocyclus mit 5 oder 7 Kettengliedern bildet, der 1 bis 4 Heteroatome ausgewählt aus Sauerstoff, Stickstoff oder Schwefel enthält, welcher Heterocyclus mindestens eine Carbonylfunktion aufweist und gegebenenfalls durch ein oder mehrere gleichartige oder verschiedenartige Halogenatome, geradkettige oder verzweigte (C₁-C₆)-Alkylgruppen, geradkettige oder verzweigte (C₁-C₆)-Alkoxygruppen oder Aminogruppen (gegebenenfalls durch eine oder zwei geradkettige oder verzweigte (C₁-C₆)-Alkylgruppen substituiert) substituiert ist.

10. Verbindung der Formel (I) nach Anspruch 1, nämlich (6S)-*N*-{4-[Amino-(hydroxyimino)methyl]-benzyl}-3-[(2,2-diphenyl-ethyl)-amino]-4-oxo-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

11. Verbindung der Formel (I) nach Anspruch 1, nämlich (6S)-*N*-{4-[Amino-(hydroxyimino)methyl]-benzyl}-4-oxo-3-{(2,2-pyridinyl)-ethyl]-amino}-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

12. Verbindung der Formel (I) nach Anspruch 1, nämlich (6S)-*N*-[2-({[Amino-(imino)methyl]-amino}-oxy)-ethyl]-3-[(2,2-diphenylethyl)-amino]-4-oxo-4, 6, 7,8-tetrahydropyrrolo[1,2-α]pyrazin-6-carboxamid, seine Isomere, seine N-Oxide sowie seine Additionssalze mit einer pharmazeutisch annehmbaren Säure oder Base.

13. Verbindung der Formel (I) nach Anspruch 1, nämlich (6S)-4-Oxo-*N*-[(3-oxo-3,4-dihydro-2*H*-benzoxazin-8-yl)-methyl]-3-{[2-(2-pyridinyl)-ethyl]-amino}-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

14. Verbindung der Formel (I) nach Anspruch 1, nämlich (6S)-4-Oxo-3-{[2-(2-pyridinyl)-ethyl]-amino}-*N*-[2-(2-pyridinyl-methoxy)-benzyl]-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

15. Verbindung der Formel (I) nach Anspruch 1, nämlich (6S)-4-Oxo-*N*-{2-[(2-oxo-3-pyrrolidinyl)-oxy]-benzyl}-3-{[2-(2-pyridinyl)-ethyl]-amino}-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

16. Verbindung der Formel (I) nach Anspruch 1, nämlich (6S)-*N*-[2-(4,5-Dihydro-1H-imidazol-2-ylmethoxy)-benzyl)-4-oxo-3-{[2-(2-pyridinyl)-ethyl]-amino}-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

17. Verbindung der Formel (I) nach Anspruch 1, nämlich (6S)-*N*-[2-(1*H-*Imidazol-2-ylmethoxy)-benzyl]-4-oxo-3-{[2-(2-pyridinyl)-ethyl]-amino]-4,6,7,8-tetrahydropyrrolo[1,2-α]pyrazin-6-carboxamid, seine Isomere, seine N-Oxide sowie seine Additionssalze mit einer pharmazeutisch annehmbaren Säure oder Base.

18. Verfahren zur Herstellung der Verbindungen der Formel (I) nach Anspruch 1, **dadurch gekennzeichnet, daß** man eine Verbindung der Formel (II): 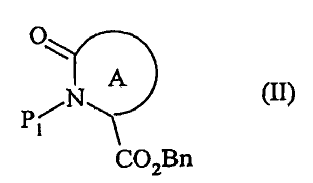 in der A die bezüglich der Formel (I) angegebenen Bedeutungen besitzt, P₁ eine Schutzgruppe der Aminofunktion darstellt und Bn eine Benzylgruppe darstellt, mit Hilfe eines geeigneten Reduktionsmittels reduziert zur Bildung der Verbindung der Formel (III): 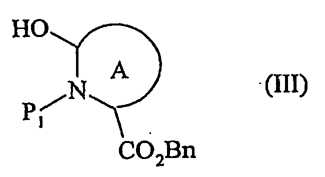 in der A, P₁ und Bn die oben angegebenen Bedeutungen besitzen,
von welcher Verbindung der Formel (III) man die Hydroxyfunktion unter Anwendung klassischer Reaktionen der organischen Chemie in die Methoxyfunktion und dann in die Cyanofunktion umwandelt, so daß man nach der Abspaltung der Schutzgruppe der Aminofunktion die verbindung der Formel (IV) erhält: 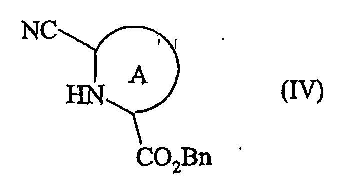 in der A und Bn die oben angegebenen Bedeutungen besitzen,
welche Verbindung der Formel (IV) man mit Oxalylchlorid umsetzt zur Bildung der Verbindung der Formel (V): 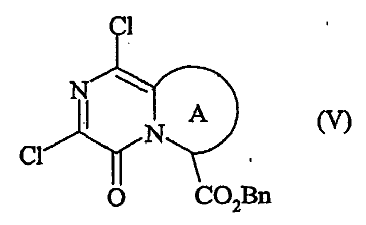 in der A und Bn die oben angegebenen Bedeutungen besitzen,
welche Verbindung der Formel (V) man mit einer Verbindung der Formel (VI):
R₁ - NH₂ (VI)
in der R₁ die bezüglich der Formel (I) angegebenen Bedeutungen besitzt, umsetzt zur Bildung der Verbindung der Formel (VII): 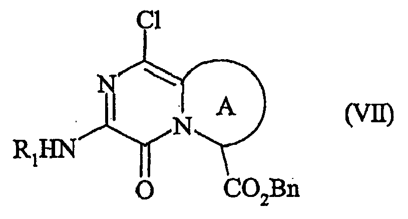 in der A, Bn und R₁ die oben angegebenen Bedeutungen besitzen,
welche Verbindung der Formel (VII) man anschließend durch katalytische Hydrierung in die Verbindung der Formel (VIII) umwandelt: 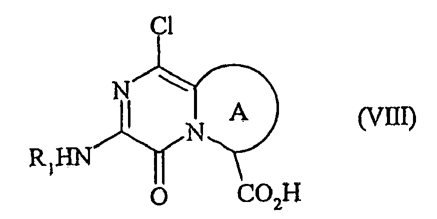 in der A und R₁ die oben angegebenen Bedeutungen besitzen,
welche Verbindung der Formel (VIII) man anschließend durch katalytische Hydrierung in alkalischem Medium in die Verbindung der Formel (IX) umwandelt: 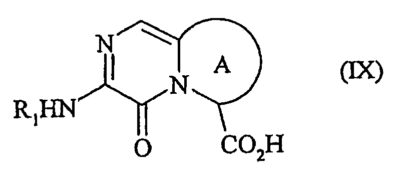 in der A und R₁ die oben angegebenen Bedeutungen besitzen,
welche Verbindung der Formel (IX) man mit einer Verbindung der Formel (X) umsetzt: 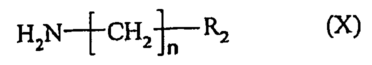 in der n und R₂ die bezüglich der Formel (I) angegebenen Bedeutungen besitzen,
so daß man nach der eventuellen Abspaltung der Schutzgruppen die Verbindung der Formel (I) erhält,
welche Verbindung der Formel (I) man gegebenenfalls in das entsprechende N-Oxid umwandelt, welche man gegebenenfalls mit Hilfe einer klassischen Reinigungsmethode reinigt, welche man gewünschtenfalls mit Hilfe einer klassischen Trennmethode in ihre Isomeren auftrennt und welche man gewünschtenfalls mit einer pharmazeutisch annehmbaren Säure oder Base in ihre Additionssalze überführt.

19. Pharmazeutische Zubereitung enthaltend als Wirkstoff eine Verbindung nach einem der Ansprüche 1 bis 17 in Kombination mit einem oder mehreren inerten, nichttoxischen und pharmazeutisch annehmbaren Hilfsstoffen.

20. Pharmazeutische Zubereitung nach Anspruch 19, nützlich als Inhibitor von mit Trypsin verwandten Serinproteasen.

21. Pharmazeutische Zubereitung nach Anspruch 20, nützlich als Inhibitor von Thrombin.
